# EUROPEAN PATENT APPLICATION

(11) **EP 2 014 276 A1**
(43) Date of publication of application: **14.01.2009**
(21) Application number: 07012049.8
(22) Date of filing: 20.06.2007
(51) Int. Cl.: A61K 8/49, A61K 8/60, A61Q 5/10, A61Q 17/04

(54) **Cosmetic compositions comprising sclareolide and hesperidin methyl chalcone**

(71) Applicant: Cognis IP Management GmbH, 40589 Düsseldorf (DE)
(72) Inventor: Moser, Philippe, 54270 Essey les Nancy (FR); Moussou, Philippe, 54510 Tomblaine (FR); Danoux, Louis, 54420 Saulxures-les-Nancy (FR); Freis, Olga, 54280 Seichamps (FR)
(74) Representative: Gittinger, Andreas

(57) **Abstract**

The present invention relates to cosmetic compositions comprising sclareolide and hesperidin methyl chalcone (HMC), which can be used especially for the tanning of skin and/or the darkening of hair and/or preventing of greying of hair. The present invention also relates to the use of agents for these purposes.

## Description

### Technical Field

The present invention relates to cosmetic compositions which can be used especially for the tanning of skin and/or the darkening of hair and/or preventing of greying of hair. The present invention also relates to the use of agents for these purposes.

### State of the Art

In modem cosmetics there is an increased demand in skin tanning agents, as tanned skin as well as dark (non grey) hair is associated with youthfulness and health. The classical method to tan skin has - for ages- been the exposure to sun light ("sun bathing"). In recent years, the knowledge on risks associated with exposure of the skin to UV radiation has increased and so has consumer's awareness of the dangers of UV radiation: UV radiation can have many damaging side effects: formation of erythrema, oxidative damages in skin, scalp or hair cells, and on structural skin or hair macromolecules, photo-ageing with loss of skin elasticity and wrinkle formation, increased incidence of skin cancer, degradation of proteins and lipids in hair, hair color fading. Thus there is a need for agents to tan skin without the risks that are linked to exposure of UV radiation.

So called "self-tanning" agents provided by the state of the art work according to the following principle: the tanning agents reacts with skin protein/amino-acids to form brown-yellow coloured substances (so called Maillard reaction). Examples for such agents include dihydroxyacetone (DHA), erythrulose, lawsone, juglone, glyceraldehyde, 6-aldo-D-fructose, hydroxymethyl-glyoxal, malealdehyde, pyruvaldehyde. Drawbacks of these tanning agents are the "artificial" skin tone that is achieved (too orange, non natural), in addition the tan/colour provided by these agents does not protect the skin against the risk of sun radiation. In addition, none of these agents is capable of preventing the greying of hair.

Unlike epidermal pigmentation, the hair pigmentation is not enhanced by UV radiation. The hair bulb melanocytes are too deep in the skin and the UV radiation does not penetrate to the hair melanogenic centers. Consequently UV-induced stimulation of hair colour is excluded. Currently known agents work as colorants, dyes or tints. These agents contain substantive dyes which are directly absorbed onto the skin or hair. One example of such a dye is henna. These agents are not without toxicological risks and the colour provided by them is not identical to the natural colour of the hair.

Thus there is an increased demand to provide safe and efficient agents which tan the skin without the risks associated with sun bathing. Preferably these agents should also provide a protection of the skin against UV radiation. At the same time there is a demand for safe and efficient agents to darken hair and/or avoid the decolouration (greying) of hair. For all these applications it is desirable that the agents are highly effective, so that they can be applied at low concentrations, thus limiting the risk of causing irritations. Naturally they should be non-toxic. In addition it is desired that these substances do not, or only to a much lower extend than products known in the market, cause an irritation of the skin/scalp onto which they are applied. At the same time these agents should be compatible with the whole array of cosmetic formulations in which they are to be applied.

Surprisingly it has been found that cosmetic compositions comprising (a) sclareolide and (b) hesperidin methyl chalcone (HMC) fulfil these demands.

From WO 02/30385 (Henkel) sclareol and/or sclareolide are known as anti-inflammatory agents in cosmetic compositions. From US 6,150,381 cosmetic compositions are known comprising as antimicrobial actives sclareol-like and sclareolide-like compounds. HMC, an abbreviation used throughout this document for hesperidin methyl chalcone, is known as pharmaceutical agent, e.g. it is disclosed in WO 02/15315 as agent to treat herpes infection or in WO 98/51291 to prepare a medicine for treating ischemia.

None of these documents discloses compositions comprising (a) and (b) nor the use of (a) or (b) according to this invention.

### Description of the Invention

The present invention is directed to cosmetic compositions comprising
(a) sclareolide
(b) hesperidin methyl chalcone (HMC)

The present invention is also directed to the use of (a) and (b) in cosmetic compositions, preferably as cosmetically active ingredients.

### Hesperidin methyl chalcone (HMC)

Hesperidin methyl chalcone, abbreviated as HMC, is a methylated chalcone derived from the flavanone-glycoside hesperidine (Hesperetin-7-O-rutinosid). The following formula displays Hesperidin methyl chalcone:

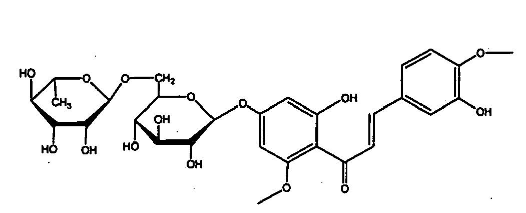

### Hesperdin Methyl Chalcone has the CAS No. CAS 24292-52-2

Hesperidin methyl chalcone means any mixture of "mono-, di- and tri-methylate".

Hesperidin methyl chalcone is commercially available, e.g. from Exquim (Barcelona, Spain) or Sigma-Aldrich (L'isle d'Abeau, France).

### Sclareolide

Sclareolide (CAS Number 564-20-5, EC Number 209-269-0) is a compound prepared by chemical modification or by biotransformation of the labdan type diterpene sclareol. Sclareol is present in stems, leaves and flowering parts of clary sage (Salvia sclarea L.). and its isolation from this source has been described (U.S. Pat. No. 3060172)

Synonyms for Sclareolide are (+)nor-ambreinolide; (3ar-(3aalpha,5abeta,9aalpha,9bbeta)) decahydro-3a,6,6,9a-tetramethyl naphth(2,1-b)furan-2(1H)-one; (3aR,5aS,9aS,9bR)- decahydro-3a,6,6,9a-tetramethyl naphtho(2,1-b)furan-2(1H)-one; 3a,4,5,5aalpha, 6,7,8,9,9a,9balpha-decahydro-3abeta,6,6,9abeta-tetramethyl naphtho(2,1-b)furan-2(1H)-one or (3aR)-(+)- sclareolide.

Sclareolide is a precursor of Ambroxan, a valuable ambergris fragrance used in perfumery. Sclareolide is commercially available and can be obtained from different suppliers as Sigma-Aldrich (L'Isle d'Abeau, France) or LGC Promochem (Molsheim, France).

### Cosmetic composition and cosmetic active concentrate

Cosmetic compositions shall mean any preparation intended to be placed in contact with the various external parts of the human body (epidermis, hair system, nails, lips and external genital organs) or with the teeth and the mucous membranes of the oral cavity with a view exclusively or mainly to cleaning them, perfuming them, changing their appearance and/or correcting body odours and/or protecting them or keeping them in good condition.

The cosmetic compositions according to the invention can for example be in the form of a hair shampoos, hair lotions, foam baths, shower baths, creams, gels, lotions, alcoholic and aqueous/alcoholic solutions, emulsions, wax/fat masses, stick preparations, powders or ointments. These compositions can also comprise, as further auxiliaries and additives, mild surfactants, oil bodies, emulsifiers, pearlescent waxes, consistency regulators, thickeners, superfatting agents, stabilizers, polymers, silicone compounds, fats, waxes, lecithins, phospholipids, UV photoprotective factors, biogenic active ingredients, antioxidants, deodorants, antiperspirants, antidandruff agents, film formers, swelling agents, insect repellents, self-tanning agents, hydrotropes, solubilizers, preservatives, perfume oils, dyes and the like.

The cosmetic compositions of the invention can comprise **sclareolide** (a) in amounts from 0,00001 to 2 weight-%, preferably 0,001 to 0,2 weight-%, based on the total weight of the final cosmetic composition.

The cosmetic compositions of the invention can comprise HMC (b) in amounts from 0,0001 to 10 weight-%, preferably 0,01 to 2 weight-%, based on the total weight of the final cosmetic composition.

The cosmetic compositions of the invention can comprise sclareolide (a) in amounts from 0,1 to 20 weight-%, based on the total weight of the final composition, this embodiment of the invention being called **"cosmetic active concentrate".**

The cosmetic composition of the invention can comprises HMC (b) in amounts from 1 - 99,9 weight-% based on the total weight of the final composition, this embodiment of the invention being called "cosmetic active concentrate".

In a preferred embodiment the invention is directed to a cosmetic active concentrate, which comprises:
- 0,1 to 20 weight-% of sclareolide (a), preferably 1 to 5 weight-%
- 1 - 99,9 weight-% HMC (b), preferably 15 to 25 weight-%.
all weight-% based on the total weight of the composition.

In one embodiment of the invention these cosmetic active concentrates can be used directly on the skin/scalp/hair, thus being cosmetic compositions themselves. In a further embodiment of the invention these cosmetic active concentrates can be used for the manufacture of cosmetic compositions, e.g. by dilution with common cosmetic ingredients, e.g. water, oil and the like.

The cosmetic compositions according to the invention can thus be prepared by adding (a) and (b) - alone or in combination- to the cosmetic composition by means knows to the man skilled in the art.

One embodiment of the invention is directed to a process of preparing a cosmetic composition, wherein the cosmetic active concentrate is diluted with solvents, if desired in the presence of solubilizers.

Suitable solvents can be selected from the group consisting of water, propylene glycol, butylene glycol, pentylene glycol and their mixtures thereof.

The solubilization can be conducted under elevated temperatures. Any known suitable solubilizers can be used, such as e.g. PEG-7-Glycerylcocoate [PEG-7 Glyceryl Cocoate is the polyethylene glycol ether of Glyceryl Cocoate (q.v.) that conforms generally to the following formula, where RCO- represents the fatty acids derived from coconut oil and x+y+z has an average value of 7.

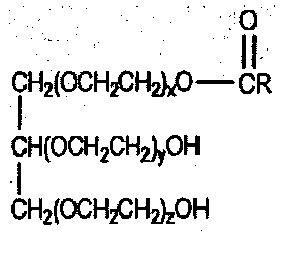

Coceth-7 [Coceth-7 is the polyethylene glycol ether of Coconut Alcohol (q.v.) that conforms to the general formula R-(OCH2CH2)ₙ-OH, wherein R represents the fatty alcohols derived from Cocos Nucifera (Coconut) Oil (q.v.) and n has an average value of 7], PPG-1-PEG-9 lauryl glycol ether, PEG-40 hydrogenated castor oil [PEG-40 Hydrogenated Castor Oil is a polyethylene glycol derivative of Hydrogenated Castor Oil (q.v.) with an average of 40 moles of ethylene oxid], PEG-20 glyceryl stearate, [PEG-20 Glyceryl Stearate is the polyethylene glycol ether of Glyceryl Stearate (q.v.) that conforms generally to the following formula, where x+y+z has an average value of 20].

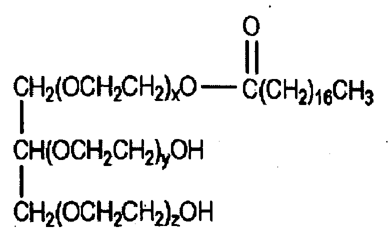

Ceteareth-12 [Ceteareth-12 is the polyethylene glycol ether of Cetearyl Alcohol (q.v.) that conforms generally to the formula R-(OCH₂CH₂)ₙ-OH wherein R represents a blend of alkyl groups derived from cetyl and stearyl alcohol and n has an average value of 12], Ceteareth-20 [Ceteareth-20 is the polyethylene glycol ether of Cetearyl Alcohol (q.v.) that conforms generally to the formula R-(OCH₂CH₂)ₙ-OH wherein R represents a blend of alkyl groups derived from cetyl and stearyl alcohol and n has an average value of 20], sodium cetearyl sulphate, or polysorbates (esters of sorbitol and sorbitol anhydrides with long chain fatty acids and condensed with ethylene oxide), such as e.g. Polysorbate-20 (Laurate Esters, approx. 20 moles EO) or Polysorbate-80 (Oleate esters, approx 80 moles EO), or mixtures thereof.

In a preferred embodiment the solubilizer is selected from the group consisting of PEG-7 Glycerylcocoate and/or Ceteareth-20, coceth-7, PPG-1-PEG-9 lauryl glycol ether, PEG-40 hydrogenated castor oil, PEG-20 glyceryl stearate, Ceteareth-12, sodium cetearyl sulphate, and/or polysorbates.

A preferred embodiment of the invention is directed to cosmetic composition comprising (a) and (b) in a weight ratio of (a) to (b) - based on the dry weight- between 100:1 to 1: 100, preferably from 50:1 to 1:50.

An especially preferred embodiment of the invention is directed to cosmetic composition comprising (a) and (b) in a weight ratio of (a) to (b) - based on the dry weight- between 1:1 and 1:25, preferably 1:5 to 1:20.

In a preferred embodiment the cosmetic compositions further comprise a skin tanning and/or hair darkening active ingredient.

Examples of further skin tanning and/or hair darkening active ingredients are substrates of tyrosinase or analogs of tyrosinase substrates such as tyrosine, Acetyl-tyrosine, palmitoyl-tyrosine or L-DOPA, stimulators of tyrosinase activity or expression such as theophylline, caffeine or isobutylmethyl xanthine, pro-opiomelanocortin peptides such as ACTH, alpha-MSH or fragments of alpha-MSH and derivatives, peptides such as Leu-Ile-Gly-Arg-NH₂ or Ser-Leu-Ile-Gly-Arg-Leu-NH₂, copper containing compounds or salts such as copper gluconate, copper glutathione complex or copper adenosine triphosphate, flavonoids such as hesperidin, neohesperidin or naringin, forskolin, diacylglycerol, agents that enhance the dendricity of melanocytes and/or that activate the transfer of melanosomes into keratinocytes such as serine proteases, agonists of the PAR-2 receptor, silymarin or silybin, purine, pyrimidine, folic acid, curcumin, extracts of chrysanthemum species, sanguisorba species, walnut extracts, urucum extracts, rhubarb extracts; Mucuna pruriens extract, juglone, lawsone, 6-aldo-D-fructose, hydroxymethyl-glyoxal, malealdehyde, pyrvaldehyde, erythrulose and dihydroxyacetone.

The composition according to the invention can also further comprise soluble melanin derivatives. Examples of commercially available soluble melanin derivatives include Melasyn-100™ from San-mar laboratories, Inc. (Elmsford, N.Y.) and MelanZe™ from Zylepsis (Ashford, Kent, United Kingdom). The compositions according to the present invention can also comprise pigments from natural sources such as for example extracts from Hedychium genus or bearberry genus, or yellow, orange and red pigments from plants containing carotenoids, or with synthetic carotenoids.

In a preferred embodiment the cosmetic compositions further comprise a substrate of tyrosinase, such as for example an ingredient selected from the group consisting of tyrosine, Acetyl-tyrosine, palmitoyl-tyrosine or L-DOPA (L-Dopamine = 3,4-Dihydroxyphenylalanine).

In a preferred embodiment the cosmetic compositions further comprise at least one ingredient selected from the group consisting of amino acids and their derivatives, forskolin, juglone, lawsone, erythrulose and dihydroxyacetone or mixtures thereof.

Suitable amino acids can be any of the 20 proteinogenic amino acids, as well as non proteinogenic amino acids, such as for example L-DOPA, omithine HCl. Derivatives of amino acids are for example N-acetylated amino acids, wherein the Aminoterminal moiety of the amino acid is acetylated, e.g. N-Acetyl-tyrosine or N-Palmitoyl-tyrosine. The acyl moiety can be of any length, preferably it comprises 1 to 16 C atoms. Amino acids can be under their free form, such as arginine, or under their Hydrochloride form, such as arginine monohydrochloride, or under their hydrate form, such as arginine monohydrate.

Examples for amino acids and their derivatives are tyrosine, Acetyl-tyrosine, palmitoyl-tyrosine, L-DOPA, Arginine, Arginine HCl, phenylalanine, omithine, omithine HCl.

In a preferred embodiment of the invention the composition further comprises Acetyl-tyrosine and Arginine or Arginine HCl.

An especially preferred embodiment of the invention is a cosmetic composition (cosmetic active concentrate) which comprises.
- 1 to 5 weight% sclareolide (a)
- 15 to 25 weight% HMC (b)
- 1 to 10 weight% of at least one amino acid or derivative

The cosmetic compositions according to the invention can preferably be used for Sclareolide (a) and/or hesperidin methyl chalcone (b), and/or their mixture as well as cosmetic compositions comprising (a) and (b) can preferably be used in cosmetics:
- as enhancer /stimulator of synthesis of melanin,
- for sunless tanning of the skin (without exposition to solar or UV radiation),
- to accelerate tanning of the skin with lower UV-irradiation,
- to homogenize at least in part skin colour when pigment spots are present on the skin, which are either lighter or darker than the surrounding area,
- to prevent and/or to reduce hair greying,
- to darken hair.

The invention is therefore directed to the use of sclareolide (a) and/or HMC (b) for the tanning (acceleration or induction) of skin.

The invention is therefore further directed to the use of sclareolide (a) and/or HMC (b) for preventing or reducing the greying of hair and/or darkening of hair.

The invention is therefore further directed to the use of sclareolide (a) and/or HMC (b) as enhancer of melanin synthesis.

The invention is further directed to a method of tanning of skin and/or darkening of hair and/or preventing of greying of hair, whereby (a) or (b) or a composition according to claims 1 to 6 is applied topically to skin, hair and/or scalp.

In one embodiment of the invention the cosmetic composition further comprises at least one surfactant.

Surface-active substances which may be present are anionic, nonionic, cationic and/or amphoteric or zwitterionic surfactants, the content of which in the compositions is usually about 1 to 70% by weight, preferably 5 to 50% by weight and in particular 10 to 30% by weight. Typical examples of anionic surfactants are soaps, alkylbenzenesulphonates, alkanesulphonates, olefinsulphonates, alkyl ether sulphonates, glycerol ether sulphonates, α-methyl ester sulphonates, sulpho fatty acids, alkyl sulphates, alkyl ether sulphates, glycerol ether sulphates, fatty acid ether sulphates, hydroxy mixed ether sulphates, monoglyceride (ether) sulphates, fatty acid amide (ether) sulphates, mono- and dialkyl sulphosuccinates, mono- and dialkyl sulphosuccinamates, sulphotriglycerides, amide soaps, ether carboxylic acids and salts thereof, fatty acid isethionates, fatty acid sarcosinates, fatty acid taurides, N-acylaminoacids, such as, for example, acyl lactylates, acyl tartrates, acyl glutamates and acyl aspartates, alkyl oligoglucoside sulphates, protein fatty acid condensates (in particular wheat-based vegetable products) and alkyl (ether) phosphates. If the anionic surfactants comprise polyglycol ether chains, these can have a conventional homologue distribution, but preferably have a narrowed homologue distribution. Typical examples of nonionic surfactants are fatty alcohol polyglycol ethers, alkylphenol polyglycol ethers, fatty acid polyglycol esters, fatty acid amide polyglycol ethers, fatty amine polyglycol ethers, alkoxylated triglycerides, mixed ethers and mixed formals, optionally partially oxidized alk(en)yl oligoglycosides and glucoronic acid derivatives, fatty acid N-alkylglucamides, protein hydrolysates (in particular wheat-based vegetable products), polyol fatty acid esters, sugar esters, sorbitan esters, polysorbates and amine oxides. If the nonionic surfactants contain polyglycol ether chains, these can have a conventional homologue distribution, but preferably have a narrowed homologue distribution. Typical examples of cationic surfactants are quaternary ammonium compounds, such as, for example, dimethyldistearylammonium chloride, and ester quats, in particular quaternized fatty acid trialkanolamine ester salts. Typical examples of amphoteric and zwitterionic surfactants are alkylbetaines, alkylamidobetaines, aminopropionates, aminoglycinates, imidazoliniumbetaines and sulphobetaines. The specified surfactants are exclusively known compounds. Typical examples of particularly suitable mild, i.e. particularly skin-compatible, surfactants are fatty alcohol polyglycol ether sulphates, monoglyceride sulphates, mono- and/or dialkyl sulphosuccinates, fatty acid isethionates, fatty acid sarcosinates, fatty acid taurides, fatty acid glutamates, α-olefinsulphonates, ether carboxylic acids, alkyl oligoglucosides, fatty acid glucamides, alkylamidobetaines, amphoacetals and/or protein fatty acid condensates, the latter preferably being based on wheat proteins.

In one embodiment of the invention the cosmetic composition further comprises at least one oil body.

Suitable oil bodies are, for example, Guerbet alcohols based on fatty alcohols having 6 to 18, preferably 8 to 10, carbon atoms, esters of linear C₆-C₂₂-fatty acids with linear or branched C₆-C₂₂-fatty alcohols and/or esters of branched C₆-C₁₃-carboxylic acids with linear or branched C₆-C₂₂-fatty alcohols, such as, for example, myristyl myristate, myristyl palmitate, myristyl stearate, myristyl isostearate, myristyl oleate, myristyl behenate, myristyl erucate, cetyl myristate, cetyl palmitate, cetyl stearate, cetyl isostearate, cetyl oleate, cetyl behenate, cetyl erucate, stearyl myristate, stearyl palmitate, stearyl stearate, stearyl isostearate, stearyl oleate, stearyl behenate, stearyl erucate, isostearyl myristate, isostearyl palmitate, isostearyl stearate, isostearyl isostearate, isostearyl oleate, isostearyl behenate, isostearyl oleate, oleyl myristate, oleyl palmitate, oleyl stearate, oleyl isostearate, oleyl oleate, oleyl behenate, oleyl erucate, behenyl myristate, behenyl palmitate, behenyl stearate, behenyl isostearate, behenyl oleate, behenyl behenate, behenyl erucate, erucyl myristate, erucyl palmitate, erucyl stearate, erucyl isostearate, erucyl oleate, erucyl behenate and erucyl erucate. Also suitable are esters of linear C₆-C₂₂-fatty acids with branched alcohols, in particular 2-ethylhexanol, esters of C₁₈-C₃₈-alkyl hydroxycarboxylic acids with linear or branched C₆-C₂₂-fatty alcohols in particular dioctyl malates, esters of linear and/or branched fatty acids with polyhydric alcohols (such as, for example, propylene glycol, dimerdiol or trimertriol) and/or Guerbet alcohols, triglycerides based on C₆-C₁₀-fatty acids, liquid mono-/di-/triglyceride mixtures based on C₆-C₁₈-fatty acids, esters of C₆-C₂₂-fatty alcohols and/or Guerbet alcohols with aromatic carboxylic acids, in particular benzoic acid, esters of C₂-C₁₂-dicarboxylic acids with linear or branched alcohols having 1 to 22 carbon atoms or polyols having 2 to 10 carbon atoms and 2 to 6 hydroxyl groups, vegetable oils, branched primary alcohols, substituted cyclohexanes, linear and branched C₆-C₂₂-fatty alcohol carbonates, such as, for example, dicaprylyl carbonate (Cetiol^{®} CC), Guerbet carbonates based on fatty alcohols having 6 to 18, preferably 8 to 10, carbon atoms, esters of benzoic acid with linear and/or branched C₆-C₂₂-alcohols (e.g. Finsolv^{®} TN), linear or branched, symmetrical or unsymmetrical dialkyl ethers having 6 to 22 carbon atoms per alkyl group, such as, for example, dicaprylyl ether (Cetiol^{®} OE), ring-opening products of epoxidized fatty acid esters with polyols, silicone oils (cyclomethicones, silicon methicone types, inter alia) and/or aliphatic or naphthenic hydrocarbons, such as, for example, squalane, squalene or dialkylcyclohexanes.

In one embodiment of the invention the cosmetic composition further comprises at least one emulsifier.

Suitable emulsifiers are, for example, nonionogenic surfactants from at least one of the following groups:
- addition products of from 2 to 30 mol of ethylene oxide and/or 0 to 5 mol of propylene oxide to linear fatty alcohols having 8 to 22 carbon atoms, to fatty acids having 12 to 22 carbon atoms, to alkylphenols having 8 to 15 carbon atoms in the alkyl group, and alkylamines having 8 to 22 carbon atoms in the alkyl radical;
- alkyl and/or alkenyl oligoglycosides having 8 to 22 carbon atoms in the alk(en)yl radical and the ethoxylated analogues thereof;
- addition products of from 1 to 15 mol of ethylene oxide to castor oil and/or hydrogenated castor oil;
- addition products of from 15 to 60 mol of ethylene oxide to castor oil and/or hydrogenated castor oil;
- partial esters of glycerol and/or sorbitan with unsaturated, linear or saturated, branched fatty acids having 12 to 22 carbon atoms and/or hydroxycarboxylic acids having 3 to 18 carbon atoms, and the adducts thereof with 1 to 30 mol of ethylene oxide;
- partial esters of polyglycerol (average degree of self-condensation 2 to 8), polyethylene glycol (molecular weight 400 to 5 000), trimethylolpropane, pentaerythritol, sugar alcohols (e.g. sorbitol), alkyl glucosides (e.g. methyl glucoside, butyl glucoside, lauryl glucoside), and polyglucosides (e.g. cellulose) with saturated and/or unsaturated, linear or branched fatty acids having 12 to 22 carbon atoms and/or hydroxycarboxylic acids having 3 to 18 carbon atoms, and the adducts thereof with 1 to 30 mol of ethylene oxide;
- mixed esters of pentaerythritol, fatty acids, citric acid and fatty alcohol and/or mixed esters of fatty acids having 6 to 22 carbon atoms, methylglucose and polyols, preferably glycerol or polyglycerol,
- mono-, di- and trialkyl phosphates, and mono-, di- and/or tri-PEG alkyl phosphates and salts thereof;
- wool wax alcohols;
- polysiloxane-polyalkyl-polyether copolymers and corresponding derivatives;
- block copolymers, e.g. polyethylene glycol-30 dipolyhydroxystearates;
- polymer emulsifiers, e.g. Pemulen grades (TR-1, TR-2) from Goodrich;
- polyalkylene glycols, and
- glycerol carbonate.

### • Ethylene oxide addition products

The addition products of ethylene oxide and/or of propylene oxide to fatty alcohols, fatty acids, alkylphenols or to castor oil are known, commercially available products. These are homologue mixtures whose average degree of alkoxylation corresponds to the ratio of the amounts of substance of ethylene oxide and/or propylene oxide and substrate with which the addition reaction is carried out. C_{12/18}-fatty acid mono- and diesters of addition products of ethylene oxide to glycerol are known as refatting agents for cosmetic preparations.

### • Alkyl and/or alkenyl oligoglycosides

Alkyl and/or alkenyl oligoglycosides, their preparation and their use are known from the prior art. They are prepared, in particular, by reacting glucose or oligosaccharides with primary alcohols having 8 to 18 carbon atoms. With regard to the glycoside radical, both monoglycosides, in which a cyclic sugar radical is glycosidically bonded to the fatty alcohol, and also oligomeric glycosides having a degree of oligomerization of up to, preferably, about 8, are suitable. The degree of oligomerization here is a statistical average value which is based on a homologue distribution customary for such technical-grade products.

### • Partial glycerides

Typical examples of suitable partial glycerides are hydroxystearic acid monoglyceride, hydroxystearic acid diglyceride, isostearic acid monoglyceride, isostearic acid diglyceride, oleic acid monoglyceride, oleic acid diglyceride, ricinoleic acid monoglyceride, ricinoleic acid diglyceride, linoleic acid monoglyceride, linoleic acid diglyceride, linolenic acid monoglyceride, linolenic acid diglyceride, erucic acid monoglyceride, erucic acid diglyceride, tartaric acid monoglyceride, tartaric acid diglyceride, citric acid monoglyceride, citric acid diglyceride, malic acid monoglyceride, malic acid diglyceride, and the technical-grade mixtures thereof which may also comprise small amounts of triglyceride as a minor product of the preparation process. Likewise suitable are addition products of 1 to 30 mol, preferably 5 to 10 mol, of ethylene oxide to said partial glycerides.

### • Sorbitan esters

Suitable sorbitan esters are sorbitan monoisostearate, sorbitan sesquiisostearate, sorbitan diisostearate, sorbitan triisostearate, sorbitan monooleate, sorbitan sesquioleate, sorbitan dioleate, sorbitan trioleate, sorbitan monoerucate, sorbitan sesquierucate, sorbitan dierucate, sorbitan trierucate, sorbitan monoricinoleate, sorbitan sesquiricinoleate, sorbitan diricinoleate, sorbitan triricinoleate, sorbitan monohydroxystearate, sorbitan sesquihydroxystearate, sorbitan dihydroxy-stearate, sorbitan trihydroxystearate, sorbitan monotartrate, sorbitan sesquitartrate, sorbitan di-tartrate; sorbitan tritartrate, sorbitan monocitrate, sorbitan sesquicitrate, sorbitan dicitrate, sorbitan tricitrate, sorbitan monomaleate, sorbitan sesquimaleate, sorbitan dimaleate, sorbitan trimaleate, and technical-grade mixtures thereof. Likewise suitable are addition products of from 1 to 30 mol, preferably 5 to 10 mol, of ethylene oxide to said sorbitan esters.

### • Polyglycerol esters

Typical examples of suitable polyglycerol esters are polyglyceryl-2 dipolyhydroxystearate (Dehymuls^{®} PGPH), polyglycerol-3 diisostearate (Lameform^{®} TGI), polyglyceryl-4 isostearate (Isolan^{®} GI 34), polyglyceryl-3 oleate, diisostearoyl polyglyceryl-3 diisostearate (Isolan^{®} PDI), polyglyceryl-3 methylglucose distearate (Tego Care^{®} 450), polyglyceryl-3 beeswax (Cera Bellina^{®}), polyglyceryl-4 caprate (Polyglycerol Caprate T2010/90), polyglyceryl-3 cetyl ether (Chimexane^{®} NL), polyglyceryl-3 distearate (Cremophor^{®} GS 32) and polyglyceryl polyricinoleate (Admul^{®} WOL 1403), polyglyceryl dimerate isostearate, and mixtures thereof. Examples of further suitable polyol esters are the mono-, di- and triesters, optionally reacted with 1 to 30 mol of ethylene oxide, of trimethylolpropane or pentaerythritol with lauric acid, coconut fatty acid, tallow fatty acid, palmitic acid, stearic acid, oleic acid, behenic acid and the like.

### • Anionic emulsifiers

Typical anionic emulsifiers are aliphatic fatty acids having 12 to 22 carbon atoms, such as, for example, palmitic acid, stearic acid or behenic acid, and dicarboxylic acids having 12 to 22 carbon atoms, such as, for example, azelaic acid or sebacic acid.

### • Amphoteric and cationic emulsifiers

Furthermore, zwitterionic surfactants can be used as emulsifiers. The term "zwitterionic surfactants" refers to those surface-active compounds which carry at least one quaternary ammonium group and at least one carboxylate and one sulphonate group in the molecule. Particularly suitable zwitterionic surfactants are the so-called betaines, such as N-alkyl-N,N-dimethylammonium glycinates, for example cocoalkyldimethylammonium glycinate, N-acylaminopropyl-N,N-dimethylammonium glycinates, for example cocoacylaminopropyl-dimethylammonium glycinate, and 2-alkyl-3-carboxymethyl-3-hydroxyethylimidazolines having in each case 8 to 18 carbon atoms in the alkyl or acyl group, and cocoacylaminoethylhydroxyethylcarboxymethyl glycinate. Particular preference is given to the fatty acid amide derivative known under the CTFA name *Cocamidopropyl Betaine*. Likewise suitable emulsifiers are ampholytic surfactants. The term "ampholytic surfactants" means those surface-active compounds which, apart from a C_{8/18}-alkyl or -acyl group, contain at least one free amino group and at least one -COOH or -SO₃H group in the molecule and are capable of forming internal salts. Examples of suitable ampholytic surfactants are N-alkylglycines, N-alkylaminopropionic acids, N-alkylaminobutyric acids, N-alkyliminodipropionic acids, N-hydroxyethyl-N-alkylamidopropylglycines, N-alkyltaurines, N-alkylsarcosines, 2-alkylaminopropionic acids and alkylaminoacetic acids having in each case about 8 to 18 carbon atoms in the alkyl group. Particularly preferred ampholytic surfactants are N-cocoalkylaminopropionate, cocoacyl-aminoethylaminopropionate and C_{12/18}-acylsarcosine. Finally, cationic surfactants are also suitable as emulsifiers, those of the ester quat type, preferably methyl-quatemized difatty acid triethanolamine ester salts, being particularly preferred.

In one embodiment of the invention the cosmetic composition further comprises at least one fat or wax.

Typical examples of fats are glycerides, i.e. solid or liquid vegetable or animal products which consist essentially of mixed glycerol esters of higher fatty acids, suitable waxes are inter alia natural waxes, such as, for example, candelilla wax, camauba wax, Japan wax, esparto grass wax, cork wax, guaruma wax, rice germ oil wax, sugarcane wax, ouricury wax, montan wax, beeswax, shellac wax, spermaceti, lanolin (wool wax), uropygial grease, ceresin, ozokerite (earth wax), petrolatum, paraffin waxes, microcrystalline waxes; chemically modified waxes (hard waxes), such as, for example, montan ester waxes, sasol waxes, hydrogenated jojoba waxes, and synthetic waxes, such as, for example, polyalkylene waxes and polyethylene glycol waxes. In addition to the fats, suitable additives are also fat-like substances, such as lecithins and phospholipids. The term lecithins is understood by the person skilled in the art as meaning those glycerophospholipids which are founded from fatty acids, glycerol, phosphoric acid and choline by esterification. Lecithins are thus also often as phosphatidylcholines (PC) in the specialist world. Examples of natural lecithins which may be mentioned are the cephalins, which are also referred to as phosphatidic acids and constitute derivatives of 1,2-diacyl-sn-glycerol-3-phosphoric acids. By contrast, phospholipids are usually understood as meaning mono- and preferably diesters of phosphoric acid with glycerol (glycerol phosphates), which are generally classed as fats. In addition, sphingosines or sphingolipids are also suitable.

In one embodiment of the invention the cosmetic composition further comprises at least one pearlescent wax.

Examples of suitable pearlescent waxes are: alkylene glycol esters, specifically ethylene glycol distearate; fatty acid alkanolamides, specifically coconut fatty acid diethanolamide; partial glycerides, specifically stearic acid monoglyceride; esters of polybasic, optionally hydroxy-substituted carboxylic acids with fatty alcohols having 6 to 22 carbon atoms, specifically long-chain esters of tartaric acid; fatty substances, such as, for example, fatty alcohols, fatty ketones, fatty aldehydes, fatty ethers and fatty carbonates, which have a total of at least 24 carbon atoms, specifically laurone and distearyl ether; fatty acids, such as stearic acid, hydroxystearic acid or behenic acid, ring-opening products of olefin epoxides having 12 to 22 carbon atoms with fatty alcohols having 12 to 22 carbon atoms and/or polyols having 2 to 15 carbon atoms and 2 to 10 hydroxyl groups, and mixtures thereof.

In one embodiment of the invention the cosmetic composition further comprises at least one consistency regulator and/or thickener.

Suitable consistency regulators are primarily fatty alcohols or hydroxy fatty alcohols having 12 to 22, and preferably 16 to 18, carbon atoms, and also partial glycerides, fatty acids or hydroxy fatty acids. Preference is given to a combination of these substances with alkyl oligoglucosides and/or fatty acid N-methylglucamides of identical chain length and/or polyglycerol poly-12-hydroxystearates. Suitable thickeners are, for example, Aerosil grades (hydrophilic silicas), polysaccharides, in particular xanthan gum, guar guar, agar agar, alginates and tyloses, carboxymethylcellulose, hydroxyethylcellulose and hydroxypropylcellulose, and also relatively high molecular weight polyethylene glycol mono- and diesters of fatty acids, polyacrylates (e.g. Carbopols^{®} and Pemulen grades from Goodrich; Synthalens^{®} from Sigma; Keltrol grades from Kelco; Sepigel grades from Seppic; Salcare grades from Allied Colloids), polyacrylamides, polymers, polyvinyl alcohol and polyvinylpyrrolidone. Bentonites, such as, for example, Bentone^{®} Gel VS 5PC (Rheox), which is a mixture of cyclopentasiloxane, disteardimonium hectorite and propylene carbonate, have also proven to be particularly effective. Also suitable are surfactants, such as, for example, ethoxylated fatty acid glycerides, esters of fatty acids with polyols such as, for example, pentaerythritol or trimethylolpropane, fatty alcohol ethoxylates having a narrowed homologue distribution or alkyl oligoglucosides, and electrolytes such as sodium chloride and ammonium chloride.

In one embodiment of the invention the cosmetic composition further comprises at least one superfatting agent.

Superfatting agents which can be used are substances such as, for example, lanolin and lecithin, and polyethoxylated or acylated lanolin and lecithin derivatives, polyol fatty acid esters, monoglycerides and fatty acid alkanolamides, the latter also serving as foam stabilizers.

In one embodiment of the invention the cosmetic composition further comprises at least one stabilizer.

Stabilizers which can be used are metal salts of fatty acids, such as, for example, magnesium, aluminium and/or zinc stearate or ricinoleate.

In one embodiment of the invention the cosmetic composition further comprises at least one polymer.

Suitable cationic polymers are, for example, cationic cellulose derivatives, such as, for example, a quaternized hydroxyethylcellulose obtainable under the name Polymer JR 400^{®} from Amerchol, cationic starch, copolymers of diallylammonium salts and acrylamides, quaternized vinylpyrrolidone-vinylimidazole polymers, such as, for example, Luviquat^{®} (BASF), condensation products of polyglycols and amines, quaternized collagen polypeptides, such as, for example, lauryldimonium hydroxypropyl hydrolyzed collagen (Lamequat^{®}L/Grünau), quaternized wheat polypeptides, polyethyleneimine, cationic silicone polymers, such as, for example, amodimethicones, copolymers of adipic acid and dimethylaminohydroxy-propyldiethylenetriamine (Cartaretins^{®}/Sandoz), copolymers of acrylic acid with dimethyldiallylammonium chloride (Merquat^{®} 550/Chemviron), polyaminopolyamides, and crosslinked water-soluble polymers thereof, cationic chitin derivatives, such as, for example, quaternized chitosan, optionally in microcrystalline dispersion, condensation products from dihaloalkyls, such as, for example, dibromobutane with bisdialkylamines, such as, for example, bis-dimethylamino-1,3-propane, cationic guar gum, such as, for example, Jaguar^{®} CBS, Jaguar^{®} C-17, Jaguar^{®} C-16 from Celanese, quaternized ammonium salt polymers, such as, for example, Mirapol^{®} A-15, Mirapol^{®} AD-1, Mirapol^{®} AZ-1 from Miranol.

Suitable anionic, zwitterionic, amphoteric and nonionic polymers are, for example, vinyl acetate-crotonic acid copolymers, vinylpyrrolidone-vinyl acrylate copolymers, vinyl acetate-butyl maleate-isobomyl acrylate copolymers, methyl vinyl ether-maleic anhydride copolymers and esters thereof, uncrosslinked polyacrylic acids and polyacrylic acids crosslinked with polyols, acrylamidopropyltrimethylammonium chloride-acrylate copolymers, octylacrylamide-methyl methacrylate-tert-butylaminoethyl methacrylate-2-hydroxypropyl methacrylate copolymers, polyvinylpyrrolidone, vinylpyrrolidone-vinyl acetate copolymers, vinylpyrrolidone-dimethylaminoethyl methacrylate-vinylcaprolactam terpolymers, and optionally derivatized cellulose ethers and silicones.

In one embodiment of the invention the cosmetic composition further comprises at least one silicone compound.

Suitable silicone compounds are, for example, dimethylpolysiloxanes, methylphenylpolysiloxanes, cyclic silicones, and amino-, fatty acid-, alcohol-, polyether-, epoxy-, fluorine-, glycoside- and/or alkyl-modified silicone compounds, which can either be liquid or in resin form at room temperature. Also suitable are simethicones, which are mixtures of dimethicones having an average chain length of from 200 to 300 dimethylsiloxane units and hydrogenated silicates.

In one embodiment of the invention the cosmetic composition further comprises at least one UV photoprotective filter.

UV photoprotective factors are, for example, to be understood as meaning organic substances (photoprotective filters) which are liquid or crystalline at room temperature and which are able to absorb ultraviolet rays and give off the absorbed energy again in the form of longer-wavelength radiation, e.g. heat. UVB filters can be oil-soluble or water-soluble. Examples of oil-soluble substances are:
- 3-benzylidenecamphor or 3-benzylidenenorcamphor and derivatives thereof, e.g. 3-(4-methylbenzylidene)camphor;
- 4-aminobenzoic acid derivatives, preferably 2-ethylhexyl 4-(dimethylamino)benzoate, 2-octyl 4-(di-methylamino)benzoate and amyl 4-(dimethylamino)benzoate;
- esters of cinnamic acid, preferably 2-ethylhexyl 4-methoxycinnamate, propyl 4-methoxycinnamate, isoamyl 4-methoxycinnamate, 2-ethylhexyl 2-cyano-3,3-phenylcinnamate (octocrylene);
- esters of salicylic acid, preferably 2-ethylhexyl salicylate, 4-isopropylbenzyl salicylate, homomenthyl salicylate;
- derivatives of benzophenone, preferably 2-hydroxy-4-methoxybenzophenone, 2-hydroxy-4-methoxy-4'-methylbenzophenone, 2,2'-dihydroxy-4-methoxybenzo-phenone;
- esters of benzalmalonic acid, preferably di-2-ethylhexyl 4-methoxybenzalmalonate;
- triazine derivatives, such as, for example, 2,4,6-trianilino(p-carbo-2'-ethyl-1'-hexyloxy)-1,3,5-triazine and octyltriazone or dioctylbutamidotriazone (Uvasorb^{®} HEB);
- propane-1,3-diones, such as, for example, 1-(4-tert-butylphenyl)-3-(4'-methoxyphenyl)propane-1,3-dione;
- ketotricyclo(5.2.1.0)decane derivatives.

Suitable water-soluble substances are:
- 2-phenylbenzimidazole-5-sulphonic acid and the alkali metal, alkaline earth metal, ammonium, alkylammonium, alkanolammonium and glucammonium salts thereof;
- sulphonic acid derivatives of benzophenones, preferably 2-hydroxy-4-methoxybenzophenone-5-sulphonic acid and its salts;
- sulphonic acid derivatives of 3-benzylidenecamphor, such as, for example, 4-(2-oxo-3-bomylidene-methyl)benzenesulphonic acid and 2-methyl-5-(2-oxo-3-bomylidene)sulphonic acid and salts thereof.

Suitable typical UV-A filters are, in particular, derivatives of benzoylmethane, such as, for example, 1-(4'-tert-butylphenyl)-3-(4'-methoxyphenyl)propane-1,3-dione, 4-tert-butyl-4'-methoxydibenzoylmethane (Parsol^{®} 1789), 1-phenyl-3-(4'-isopropylphenyl)propane-1,3-dione, and enamine compounds. The UV-A and UV-B filters can of course also be used in mixtures. Particularly favourable combinations consist of the derivatives of benzoylmethane, e.g. 4-tert-butyl-4'-methoxydi-benzoylmethane (Parsol^{®} 1789) and 2-ethylhexyl 2-cyano-3,3-phenylcinnamate (octocrylene) in combination with esters of cinnamic acid, preferably 2-ethylhexyl 4-methoxycinnamate and/or propyl 4-methoxycinnamate and/or isoamyl 4-methoxycinnamate. Advantageously, such combinations are combined with water-soluble filters such as, for example, 2-phenylbenzimidazole-5-sulphonic acid and their alkali metal, alkaline earth metal, ammonium, alkylammonium, alkanolammonium and glucammonium salts.

As well as said soluble substances, insoluble light protection pigments, namely finely dispersed metal oxides or salts, are also suitable for this purpose. Examples of suitable metal oxides are, in particular, zinc oxide and titanium dioxide and also oxides of iron, zirconium, silicon, manganese, aluminium and cerium, and mixtures thereof. Salts which may be used are silicates (talc), barium sulphate or zinc stearate. The oxides and salts are used in the form of the pigments for skincare and skin-protective emulsions and decorative cosmetics. The particles here should have an average diameter of less than 100 nm, preferably between 5 and 50 nm and in particular between 15 and 30 nm. They can have a spherical shape, but it is also possible to use particles which have an ellipsoidal shape or a shape deviating in some other way from the spherical form. The pigments can also be surface-treated, i.e. hydrophilicized or hydrophobicized. Typical examples are coated titanium dioxides, such as, for example, titanium dioxide T 805 (Degussa) or Eusolex^{®} T2000 (Merck). Suitable hydrophobic coating agents are here primarily silicones and, specifically in this case, trialkoxyoctylsilanes or simethicones. In sunscreens, preference is given to using so-called micro- or nanopigments. Preference is given to using micronized zinc oxide.

In one embodiment of the invention the cosmetic composition further comprises at least one biogenic active ingredient and/or antioxidant.

Biogenic active ingredients are understood as meaning, for example, tocopherol, tocopherol acetate, tocopherol palmitate, ascorbic acid, (deoxy)ribonucleic acid and fragmentation products thereof, β-glucans, retinol, bisabolol, allantoin, phytantriol, panthenol, AHA acids, amino acids, ceramides, pseudoceramides, essential oils, plant extracts, such as, for example, prunus extract, bambara nut extract and vitamin complexes.

Antioxidants interrupt the photochemical reaction chain which is triggered when UV radiation penetrates the skin. Typical examples thereof are amino acids (e.g. glycine, histidine, tyrosine, tryptophan) and derivatives thereof, imidazoles (e.g. urocanic acid) and derivatives thereof, peptides, such as D,L-camosine, D-camosine, L-camosine and derivatives thereof (e.g. anserine), carotenoids, carotenes (e.g. α-carotene, β-carotene, lycopene) and derivatives thereof, chlorogenic acid and derivatives thereof, lipoic acid and derivatives thereof (e.g. dihydrolipoic acid), aurothioglucose, propylthiouracil and other thiols (e.g. thioredoxin, glutathione, cysteine, cystine, cystamine and the glycosyl, N-acetyl, methyl, ethyl, propyl, amyl, butyl and lauryl, palmitoyl, oleyl, γ-linoleyl, cholesteryl and glyceryl esters thereof) and salts thereof, dilauryl thiodipropionate, distearyl thiodipropionate, thiodipropionic acid and derivatives thereof (esters, ethers, peptides, lipids, nucleotides, nucleosides and salts), and sulphoximine compounds (e.g. buthionine sulphoximines, homocysteine sulphoximine, buthionine sulphones, penta-, hexa-, heptathionine sulphoximine) in very low tolerated doses (e.g. pmol to µmol/kg), and also (metal) chelating agents (e.g. α-hydroxy fatty acids, palmitic acid, phytic acid, lactoferrin), α-hydroxy acids (e.g. citric acid, lactic acid, malic acid), humic acid, bile acid, bile extracts, bilirubin, biliverdin, EDTA, EGTA and derivatives thereof, unsaturated fatty acids and derivatives thereof (e.g. γ-linolenic acid, linoleic acid, oleic acid), folic acid and derivatives thereof, ubiquinone and ubiquinol and derivatives thereof, vitamin C and derivatives (e.g. ascorbyl palmitate, Mg ascorbyl phosphate, ascorbyl acetate), tocopherols and derivatives (e.g. vitamin E acetate), vitamin A and derivatives (vitamin A palmitate), and coniferyl benzoate of gum benzoin, rutic acid and derivatives thereof, α-glycosylrutin, ferulic acid, furfurylideneglucitol, carnosine, butylhydroxytoluene, butylhydroxyanisole, nordihydroguaiacic acid, nordihydroguaiaretic acid, trihydroxybutyrophenone, uric acid and derivatives thereof, mannose and derivatives thereof, superoxide dismutase, zinc and derivatives thereof (e.g. ZnO, ZnSO₄) selenium and derivatives thereof (e.g. selenomethionine), stilbenes and derivatives thereof (e.g. stilbene oxide, trans-stilbene oxide) and the derivatives (salts, esters, ethers, sugars, nucleotides, nucleosides, peptides and lipids) of said active ingredients which are suitable according to the invention.

In one embodiment of the invention the cosmetic composition further comprises at least one antimicrobial agent and/or preservative.

Suitable antimicrobial agents are, in principle, all substances effective against gram-positive bacteria, such as, for example, 4-hydroxybenzoic acid and its salts and esters, N-(4-chlorophenyl)-N'-(3,4-dichlorophenyl)urea, 2,4,4'-trichloro-2'-hydroxydiphenyl ether (triclosan), 4-chloro-3,5-dimethylphenol, 2,2'-methylenebis(6-bromo-4-chlorophenol), 3-methyl-4-(1-methylethyl)phenol, 2-benzyl-4-chlorophenol, 3-(4-chlorophenoxy)-1,2-propanediol, 3-iodo-2-propynyl butylcarbamate, chlorhexidine, 3,4,4'-trichlorocarbanilide (TTC), antibacterial fragrances, thymol, thyme oil, eugenol, oil of cloves, menthol, mint oil, famesol, phenoxyethanol, glycerol monocaprate, glycerol monocaprylate, glycerol monolaurate (GML), diglycerol monocaprate (DMC), salicylic acid N-alkylamides, such as, for example, N-octylsalicylamide or N-decylsalicylamide.
Suitable preservatives are, for example, phenoxy ethanol, formaldehyde solution, parabens, pentanediol or sorbic acid, and the silver complexes known under the name Surfacins^{®}, and also the other classes of substance listed in Annex 6, Part A and B of the Cosmetics Directive.

In one embodiment of the invention the cosmetic composition further comprises at least one film former.

Customary film formers are, for example, chitosan, microcrystalline chitosan, quaternized chitosan, polyvinylpyrrolidone, vinylpyrrolidone-vinyl acetate copolymers, polymers of the acrylic acid series, quaternary cellulose derivatives, collagen, hyaluronic acid and salts thereof, and similar compounds.

In one embodiment of the invention the cosmetic composition further comprises at least one swelling agent.

The swelling agents for aqueous phases may be montmorillonites, clay mineral substances, Pemulen, and alkyl-modified Carbopol grades (Goodrich). Other suitable polymers and swelling agents are given in the review by R. Lochhead in **Cosm. Toil. 108, 95 (1993).**

In one embodiment of the invention the cosmetic composition further comprises at least one hydrotrophic agent.

To improve the flow behaviour, it is also possible to use hydrotropic agents, such as, for example, ethanol, isopropyl alcohol, or polyols. Polyols which are suitable here preferably have 2 to 15 carbon atoms and at least two hydroxyl groups. The polyols can also contain further functional groups, in particular amino groups, or be modified with nitrogen. Typical examples are
- glycerol;
- alkylene glycols, such as, for example, ethylene glycol, diethylene glycol, propylene glycol, butylene glycol, hexylene glycol, and polyethylene glycols with an average molecular weight of from 100 to 1 000 daltons;
- technical-grade oligoglycerol mixtures with a degree of self-condensation of from 1.5 to 10, such as, for example, technical-grade diglycerol mixtures with a diglycerol content of from 40 to 50% by weight;
- methylol compounds, such as, in particular, trimethylolethane, trimethylolpropane, trimethylolbutane, pentaerythritol and dipentaerythritol;
- lower alkyl glucosides, in particular those having 1 to 8 carbon atoms in the alkyl radical, such as, for example, methyl and butyl glucoside;
- sugar alcohols having 5 to 12 carbon atoms, such as, for example, sorbitol or mannitol,
- sugars having 5 to 12 carbon atoms, such as, for example, glucose or sucrose;
- amino sugars, such as, for example, glucamine;
- dialcohol amines, such as diethanolamine or 2-amino-1,3-propanediol.

The total amount of further components can be 1 to 50% by weight, preferably 5 to 40% by weight, based on the compositions. The compositions can be prepared by customary cold or hot processes; preference is given to using the phase-inversion temperature method.

### Examples

Hesperidin methyl chalcone used in the examples was obtained from Sigma Aldrich (CAS Number 24292-52-2), Sclareolide was obtained from Sigma Aldrich (CAS No CAS Number 564-20-5).

### Example 1: Melanogenesis stimulation assay for sclareolide and hesperidin methylchalcone

Melanocytes (B16 cell line) were inoculated in standard medium of cell culture with foetal calf serum (FCS). After an incubation of 3 day at 37°C and CO2=5%, growth medium was exchanged for standard medium with a range of concentrations for each active ingredient to be tested and a control without ingredient. After an incubation of 3 days, the level of melanin was measured by recording the optical density at 475 nm. After washing the cells by a balanced salt solution, and homogenisation in a solution of 0.1 M NaOH, the number of viable cells was determined by evaluation of the level of cellular proteins (Bradford's method). The results are expressed in % against control (cell culture medium without compound) as a mean +/- SEM (Standard Error of Mean) on 2 or 3 assays, each in triplicate.

**Table 1. Rate of cellular proteins & melanin in % / control (mean +/-SEM on 3 assays in triplicate):**

| | Dose % (w/v) | Protein level | Melanin level |
|---|---|---|---|
| Control | - | 100+/-0 | 100+/-0 |
| Sclareolide | 0.0003 | 89 +/-11 | 174 +/-55 |
| | 0.001 | 83 +/-17 | 286 +/-83 |
| Hesperidin methylchalcone | 0.01 | 110+/-2 | 189 +/-32 |
| | 0.03 | 101+/-13 | 381+/-16 |

The results demonstrated that the compounds have increased the rate of melanin synthesis in melanocytes, without cell toxicity.

### Example 2. Melanogenesis stimulation assay for combination of sclareolide and hesperidin methylchalcone

Melanocytes (B16 cell line) were inoculated in standard medium of cell culture with foetal calf serum (FCS). After an incubation for 3 days at 37°C and CO₂=5%, growth medium was exchanged for standard medium with a range of concentrations for each active ingredient to be tested and a control without ingredient. Combinations of active ingredients were tested on the same cultures, in parallel with active ingredients alone. After an incubation of 3 days, the cells were washed by a balanced salt solution, and homogenised in a solution of 0.1 M NaOH. The number of viable cells was determined by evaluation of the level of cellular proteins (Bradford's method) and the level of intracellular melanin was measured by recording the optical density at 475 nm.

The results are expressed in % against control (cell culture medium without compound) as a mean +/-SEM (Standard Error of Mean) on 5 assays, each in triplicate.

**Table 2: rate of melanin in % / control (mean +/-SEM on triplicates):**

| | | Hesperidin methylchalcone | | |
|---|---|---|---|---|
| | | Dose % (w/v) | | |
| | | Control | 0.00025 | 0.00050 |
| Sclareolide | Control | 100+/-0 | 112+/-8 | 110+/-9 |
| | 0.000025 | 116+/-7 | 135+/-8 | nt |
| Dose % (w/v) | 0.000050 | 120+/-6 | nt | 159+/-9 |

| | | | | |
|---|---|---|---|---|
| nt: not tested | | | | |

At very low doses sclareolide has increased the rate of melanin synthesis, whereas at very low doses hesperidin methyl chalcone did not display a significant efficacy. When associated at the same doses, the combination of sclareolide and hesperidin methyl chalcone has resulted in an efficacy higher than each component alone, demonstrating a synergistic effect.

**Table 3: Amount of cellular proteins in % / control (mean +/-SEM on triplicates):**

| | | Hesperidin methylchalcone | | |
|---|---|---|---|---|
| | | | | |
| | | Dose % (w/v) | | |
| | | Control | 0.00025 | 0.00050 |
| Sclareolide | Control | 100+/-0 | 109+/-15 | 103+/-10 |
| | 0.000025 | 97+/-18 | 94+/-5 | nt |
| Dose % (w/v) | 0.000050 | 106+/-10 | nt | 91+/-8 |

| | | | | |
|---|---|---|---|---|
| nt: not tested | | | | |

Sclareolide or hesperidine methyl chalcone tested separately or mixed, have not distinctly decreased the amount of cellular proteins and therefore they do not show any toxic effects at these concentrations.

### Example 3 Cosmetic compositions

### Cream formulation 1

| Ingredient [INCI] | % by weight |
|---|---|
| Emulgade® SE-PF ⁽²⁾ | 5.00 |
| [Glyceryl Stearate (and) Ceteareth-20 (and) Ceteareth-12 (and) Ceteareth Alcohol (and) Cetyl Palmitate] | |
| Lanette®22 ⁽²⁾ [Behenyl alcohol] | 2.00 |
| Cegesoft® C24 ⁽²⁾ (Ethylhexylpalmitate) | 4.00 |
| Cetiol® PGL ⁽²⁾ [Hexyldecanol, Hexyldecyl Laurate] | 4.00 |
| Eumulgin® B2 ⁽²⁾ [Ceteareth- 20] | 0.40 |
| DC 200-350cts ⁽³⁾ [Dimethicone] | 1.00 |
| PEG7 Glyceryl cocoate | 1.00 |
| Butylene glycol | 1.00 |
| Cosmedia® SP ⁽²⁾ [Sodium Polyacrylate] | 0.80 |
| Plantapon® ACG 35 ⁽²⁾ [Disodium Cocoyl Glutamate] | 0.80 |
| Elestab® 50J ⁽¹⁾ [Chlorphensin and Methylparaben] | 0.40 |
| Glycerine | 4.00 |
| Sclareolide | 0.20 |
| Hesperidin methylchalcone | 1.00 |
| Acetyl-tyrosine | 0.10 |
| Arginine hydrochloride | 0.05 |
| Deionized water | add up to 100 |

### Cream formulation 2

| Ingredient [INCI] | % by weight |
|---|---|
| Eumulgin® VL 75 ⁽²⁾ | 4,00 |
| [Lauryl Glucoside (and) Polyglyceryl-2 Dipolyhydroxystearate (and) Glycerin] | |
| Lanette® 22 ⁽²⁾ [Behenyl alcohol] | 2.00 |
| Cetiol® 868 ⁽²⁾ [Ethylhexyl Stearate] | 4.00 |
| Cetiol® OE ⁽²⁾ [Dicaprylylether] | 2.00 |
| Cetiol® PGL ⁽²⁾ [Hexyldecanol, Hexyldecyl Laurate] | 4.00 |
| DC 200-350cts ⁽³⁾ [Dimethicone] | 1.00 |
| Cosmedia® SP ⁽²⁾ [Sodium Polyacrylate] | 1.00 |
| Elestab® 388 ⁽¹⁾ | 2.50 |
| [Propylene Glycol and Phenoxyethanol and Chlorphensin and Methylparaben] | |
| Keltrol® CGT⁽⁴⁾ [Xanthan Gum] | 0.25 |
| Glycerin | 3.00 |
| Plantapon® ACG 35⁽²⁾ [Disodium Cocoyl Glutamate] | 0.80 |
| Hesperidin methyl chalcone | 2.00 |
| Deionized water | add up to 100 |

### Cream formulation 3

| Ingredient [INCI] | % by weight |
|---|---|
| Emulgade® NLB⁽²⁾ | 4.00 |
| [Steareth-2 (and) Ceteareth-12 (and) Stearyl Alcohol (and) Ceteareth-20 (and) Distearyl Ether] | |
| Lanette® 22⁽²⁾ [Behenyl alcohol] | 1.50 |
| Cutina® PES⁽²⁾ [Pentaerythrityl Distearate] | 1.00 |
| Cegesoft® C24⁽²⁾ (Ethylhexylpalmitate) | 4.00 |
| Cetiol® LC⁽²⁾ [Coco caprylate/caprate] | 3.00 |
| Cetiol® CC⁽²⁾ [Dicaprylyl Carbonate] | 2.00 |
| Cosmedia® SP⁽²⁾ [Sodium Polyacrylate] | 0.70 |
| DC 200-350cts ⁽³⁾ [Dimethicone] | 1.00 |
| Glycerin | 3.00 |
| Elestab® 388 ⁽¹⁾ | 2.50 |
| [Propylene Glycol and Phenoxyethanol and Chlorphensin and Methylparaben] | |
| Eumulgin® SG⁽²⁾ [Sodium Stearoyl Glutamate] | 1.00 |
| Scalreolide | 0.50 |
| Deionized water | add up to 100 |

### Hair lotion

| Ingredient [INCI] | % by weight |
|---|---|
| Ethanol | 18.20 |
| Elestab® 50J ⁽¹⁾ [Chlorphensin and Methylparaben] | 0.30 |
| Hesperidin methylchalcone | 0.50 |
| Sclareolide | 0.05 |
| Citric Acid 10% | qsf pH 5.5 |
| Deionized water | add up to 100 |

### Hair balm

| Ingredient [INCI] | % by weight |
|---|---|
| Dehyquart® C 4046 ⁽²⁾ | 3.50 |
| [Cetearyl Alcohol and Dipalmitoylethyl Hydroxyethylmonium Methosulfate and Ceteareth-20] | |
| Eumulgin® B2 ⁽²⁾ [Ceteareth-20] | 0.40 |
| Lanette® O ⁽²⁾ [Cetearyl Alcohol] | 2.50 |
| Cetiol® J 600 ⁽²⁾ [Oleyl Erucate] | 3.00 |
| Butylene glycol | 0.20 |
| PEG7 Glyceryl cocoate | 0.10 |
| Elestab® 388⁽¹⁾ | 2.50 |
| [Propylene Glycol and Phenoxyethanol and Chlorphensin and Methylparaben] | |
| Glycerin | 2.00 |
| Cosmedia® Guar C 261⁽²⁾ [Guar Hydroxypropyltrimonium Chloride] | 0.15 |
| Sclareolide | 0.50 |
| Hesperidin methylchalcone | 0.10 |
| Acetyl-tyrosine | 0.05 |
| Arginine hydrochloride | 0.05 |
| Deionized water | add up to 100 |

Suppliers:
(1) Laboratoires Sérobiologiques. (2) Cognis, (3) Dow Coming, (4) Kelco

## Claims

1. **Cosmetic composition** comprising
(a) sclareolide
(b) hesperidin methyl chalcone (HMC)

2. **Cosmetic composition** according to any preceding claim, wherein the weight ratio of (a) to (b) - based on the dry weight- is between 100:1 to 1:100, preferably from 50:1 to 1:50.

3. **Cosmetic composition** according to any preceding claim, which further comprises a skin tanning and/or hair darkening active ingredient.

4. **Cosmetic composition** according to claim 3, wherein the further active ingredient is a substrate of tyrosinase.

5. **Cosmetic composition** according to any preceding claim further comprising at least one ingredient selected from the group consisting of amino acids and their derivatives, forskolin, juglone, lawsone, erythrulose and dihydroxyacetone.

6. **Cosmetic composition** according to claim 5, wherein the amino acids and their derivatives are selected from the group consisting of tyrosine, Acetyl-tyrosine, palmitoyl-tyrosine, L-DOPA, Arginine, Arginine HCl, phenylalanine, omithine, omithine HCl.

7. **Cosmetic use** of (a) and/or (b) for the tanning (acceleration or induction) of skin.

8. **Cosmetic use** of (a) and/or (b) for preventing or reducing the greying of hair and/or darkening of hair.

9. **Cosmetic use** of (a) and/or (b) as enhancer of melanin synthesis.

10. **Method** of tanning of skin and/or darkening of hair and/or preventing of greying of hair, whereby (a) or (b) or a composition according to claims 1 to 6 is applied topically to skin, hair or scalp.
